# EUROPEAN PATENT APPLICATION

(11) **EP 2 905 340 A1**
(43) Date of publication of application: **12.08.2015**
(21) Application number: 14305169.6
(22) Date of filing: 07.02.2014
(51) Int. Cl.: C12P 7/16, C12P 7/18

(54) **Biological coproduction of 1,3-propanediol and n-butanol**

(71) Applicant: ARKEMA FRANCE, 92700 Colombes (FR); Centre National de la Recherche Scientifique (CNRS), 75794 Paris Cedex 16 (FR)
(72) Inventor: Zeng, An-Ping, 21224 Rosengarten (DE); Sabra, Wael, 21614 Buxtehude (DE); Groeger, Christin, 21073 Hamburg (DE); Dubois, Jean-Luc, 69390 Millery (FR)
(74) Representative: Cabinet Plasseraud

(57) **Abstract**

The present invention relates to a method for the anaerobic coproduction of 1,3-propanediol and n-butanol comprising the successive steps of culturing a *Clostridium* species strain in a fermentation broth comprising at least glycerol as a source of carbon, thereby producing n-butanol and 1,3-propanediol, recovering n-butanol and recovering 1,3-propanediol from the fermentation broth, characterized in that a reducing agent other than H₂ is added to the fermentation broth and the fermentation broth is sparged with a gas selected from H₂, N₂, CO₂ and their mixtures, or the fermentation broth is sparged with a gas comprising CO₂, H₂ and N₂.

## Description

The research leading to these results has received funding from the European Union Seventh Framework Programme (FP7/2007-2013) under grant agreement n°241718 EuroBioRef.

### FIELD OF THE INVENTION:

The present invention relates to a method for the anaerobic coproduction of 1,3-propanediol and n-butanol.

### BACKGROUND OF THE INVENTION:

n-butanol and 1,3-propanediol (1,3-PDO) are two important chemicals. Butanol is an important industrial solvent and a bulk chemical used for the synthesis of various chemicals and is widely recognized as a better fuel than ethanol. Butanol has several advantages over ethanol for fuel. While it can be made from the same feedstocks as ethanol, unlike ethanol it is compatible with gasoline and diesel at any ratio. Butanol can also be used alone as a pure fuel in existing cars without modifications. 1-butanol (also called n-butanol) is also a common chemical compound used in the synthesis of butylacrylate, butylacetate, 1,1-dibutoxybutane, 1,1-dibutoxymethane (butylal), acetone dibutyl acetal and glycol ether formulations for example. 1,3-propanediol is a monomer useful in the manufacture of polyester compounds, polyurethanes, polytrimethylene carbonate (PTMC) and polytrimethylene terephthalate (PTT), which may be used in the production of textile fibers.
Worldwide, there are large interests and efforts to produce n-butanol and 1,3-PDO from renewable biomass material by using dedicated fermentation technologies with microorganisms.
Both the microbial production processes for 1,3-PDO and n-butanol have been industrially realized and commercialized. Conventionally, one specific fermentation process is used to produce either 1,3-PDO or n-butanol.
A fermentation process using *Clostridium pasteurianum* for producing both 1,3-PDO and n-butanol from glycerol is known (H. Biebl: J. Ind. Microbial Biotechnol. 2001, 27, 18-26).
However, the yield of this kind of fermentation is limited by the toxicity of the substrate and/or by the inhibitory effect of butanol thus produced on the growth of the bacteria and thus on the production of 1,3-PDO. In order to reduce the toxicity of crude glycerol, Jensen et al. have used activated carbon stone and mutant strain (Jensen TO. et al., AMB Express. 2012 Aug 17;2(1):44 and Jensen TO. et al., J Ind Microbiol Biotechnol. 2012 May;39(5):709-17). Moreover, it has been shown that the inhibitory effect of butanol could be prevented either by using a genetically-modified bacteria (US-8,236,994) or by gas stripping of butanol from the fermentor (Jensen TO. et al., J Ind Microbiol Biotechnol. 2012 May;39(5):709-17).
In addition to the toxicity of n-butanol, this fermentation process is also very unstable, with large fluctuation of the product spectrum under different conditions, with n-butanol being the main product mostly. The maximum n-butanol concentration reached so far is 17 g/L, the 1,3-PDO concentration is in the range of 2,5 g/L (H. Biebl: J. Ind. Microbial Biotechnol. 2001, 27, 18-26).

In view of the foregoing, there is still a need to design a stable process reproducible on a large scale which may produce both n-butanol and 1,3-PDO with high yields, i.e. typically more than 15g/L each in a single run.

### SUMMARY OF THE INVENTION:

Now, the applicants have found that the above needs can be satisfied by controlling the redox potential during the fermentation process.
Specifically, the redox potential can be maintained to an optimal level by adding a reducing agent to the fermentation broth and/or by sparging with a gas having a certain composition. The gas-sparging can be carried out from the beginning of the fermentation or after a certain time of fermentation in form of an in situ gas stripping or by combining both measures.
The control of redox potential is thus favorable for an optimized coproduction of 1,3-PDO and n-butanol and leads to a reproducible and stable production process.

A subject of the present invention is therefore a method for the anaerobic coproduction of 1,3-propanediol (1,3-PDO) and n-butanol comprising the successive steps of:
(a) culturing a *Clostridium* species in a fermentation broth comprising at least glycerol as a source of carbon, thereby producing n-butanol and 1,3-propanediol,
(b) recovering n-butanol and
(c) recovering 1,3-propanediol from the fermentation broth, characterized in that:
   - a reducing agent other than H₂ is added to the fermentation broth and the fermentation broth is sparged with a gas selected from H₂, N₂, CO₂ and their mixtures,
      or
   - the fermentation broth is sparged with a gas comprising CO₂, H₂ and N₂.

This process allows maintaining the redox potential of the fermentation broth between -800mV and -100mV, preferably between -600 to -400 mV.

### DETAILED DESCRIPTION OF THE INVENTION

The first step of the method according to the present invention comprises culturing a *Clostridium* species in a fermentor comprising at least glycerol as a source of carbon, under anaerobic conditions, thereby producing n-butanol and 1,3-propanediol.

In one embodiment, the method of the invention comprises a preliminary step of feeding the fermentor in which the culture is run with a culture medium.
The culture medium comprises a source of carbon.
The source of carbon comprises at least glycerol, which may be pure or crude glycerol, and optionally glucose or biomass hydrolysates
Preferably, the source of carbon is constituted of glycerol, as it was observed that the co-production of acids, such as butyric acid and acetic acid, is lowered in that case.

The culture medium may comprise one or more reducing agents other than H₂. The reducing agent may be for example cystein, methionine or sodium sulphite. Preferably, the reducing agent is cystein.
The culture medium may also comprise one or more additives, which may be selected from the group consisting of at least one source of iron, at least one source of phosphate, at least one source of nitrogen, one or more minerals, one or more buffers, one or more triggers, and their mixtures.
The source of iron may be for example FeSO₄.
The source of phosphate may be for example KH₂PO₄ and/or K₂HPO₄.
The source of nitrogen may be for example a yeast extract, ammonia or urea.

In one embodiment, the method of the invention comprises a step of inoculating the *Clostridium* species into the fermentor.
Preferably, the *Clostridium* species is selected from the group consisting of a *Clostridium pasteurianum, Clostridium butyricum* and *Clostridium diolis.* More preferably, the *Clostridium species* is a *Clostridium pasteurianum.* Most preferably, the *Clostridium species* is a *Clostridium pasteurianum* DSM 525.

A fermentation broth is thus obtained, which corresponds to the mixture of the culture medium, the bacteria, the products of fermentation and all the other constituents held in the fermentor in which the *Clostridium* strain is cultured.
In a first embodiment, wherein a reducing agent is present in the fermentation broth, the anaerobic condition with a favourable redox potential is created by gassing the fermentor with a gas containing at least one gas selected from CO₂, H₂, N₂ and their mixtures, preferably with the fermentation gas.
As used therein, the fermentation gas is a gas produced by culturing the *Clostridium* species.
Indeed, the fermentation leads to the production of gases, mainly CO₂, and H₂ which may be used for sparging. N₂ may also be present due to the presence of air at the beginning of the fermentation, or may be added from an external air separation unit. Moreover, CO₂ may be added from another fermentor. The use of the gases from the fermentation for sparging reduces the cost of the method and leads to better fermentation behaviour.
At the beginning of the culture, this fermentation gas may be collected from a previous fermentation or from one or several neighbouring fermentor(s).
During the fermentation, the fermentation gas may be the own gas produced by the fermentation.

In a second embodiment, wherein a reducing agent is not present in the fermentation broth, the required redox potential is obtained by sparging a a mixture of N₂, H₂ and CO₂ into the fermentation broth. These gases may come at least partly from the fermentation, as explained above.

In a preferred embodiment, the step of sparging the fermentation broth with a gas comprises a step of gas stripping in order to continuously remove butanol from the fermentation broth, as will be explained hereafter.
"Stripping" as used herein means the action of transferring all or part of a volatile component from a liquid stream into a gaseous stream.
In one embodiment, at the beginning of the fermentation, the fermentation broth is sparged with a gas, preferably a gas collected from a previous fermentation, then, during the fermentation, the fermentation broth is sparged through gas stripping using own fermentation gas.

The fermentation is run, preferably, at a temperature from 34°C to 65°C, more preferably from 34°C to 37°C, most preferably at 35°C.
In one embodiment, the pH of the fermentation broth is kept constant at a value comprised between 5 and 7, preferably from 5.5 to 6.5, more preferably 6.
The pH may be kept by the addition of a base, preferably a strong base, for example KOH, ammonia, urea or NaOH, preferably KOH, more preferably KOH at 2.5M.
Moreover, the fermentation broth is usually kept under stirring.

In the second step of the method according to this invention, the n-butanol produced by the fermentation is separated from the fermentation broth. In one embodiment, the step of recovering n-butanol is started when the concentration of n-butanol in the fermentation broth reaches a selected value comprised between 4 and 6 g/L, preferably 5 g/L.
Starting the recovering of n-butanol not from the beginning of the fermentation, but at a later stage ensures a better growth of the bacteria through the whole process.
n-butanol may be recovered from the fermentation broth for example by gas stripping, solvent extraction and other techniques well known to the skilled artisan.
Preferably, n-butanol is recovered by passing a flow of stripping gas through the fermentation broth. This stripping gas may be any of the sparging gases and gas mixtures described previously. The fermentation gas is preferably used as a stripping gas. The n-butanol enriched gas thus formed is called herein a n-butanol enriched stripping gas or enriched stripping gas.
Thus, this in situ gas stripping allows the removal of n-butanol from the fermentation broth, in order to lower its concentration to a non-toxic level, and possibly also reducing the redox potential.

n-butanol is directly removed from the fermentor; there is no need to transfer the fermentation broth to an additional unit in order to operate the gas stripping. In situ separation of butanol also facilitates down-stream processing. Gas stripping results in a pre-purified mixture of butanol and water which may be easily separated.
The stripping gas flow rate can be adjusted in such a way as to keep the butanol in the fermentation broth below 5g/L.

The flow of stripping gas passes through the fermentation broth, thus forming a n-butanol enriched stripping gas, which may also contain ethanol but is usually deprived of butyric acid and acetone.
The n-butanol from the enriched stripping gas is recovered by condensation in a condenser.
In one embodiment, the condenser is cooled at from 0°C to room temperature, preferably from 0°C to 5°C, more preferably at 1°C by a cooling liquid such as a glycolic solution, or ice slurry.

In one embodiment, the n-butanol enriched stripping gas flows downwardly through the condenser. In this situation, the n-butanol enriched stripping gas enters at or near the top of the condenser and exits at or near the bottom of the condenser. Thus, there is a co-flow direction of the n-butanol enriched stripping gas with the condensate.
On the contrary, the cooling liquid flows counter-currently to the n-butanol enriched stripping gas. In the latter situation, the cooling liquid flows upwardly through the condenser. The cooling liquid thus enters at or near the bottom of the condenser and exits at or near the top of the condenser.
Alternatively the gas may flow upwards, and the cooling medium downwards and the head of the condenser column may be equipped with a demister so as to avoid droplets falling back to the fermentor.

In one embodiment, the condensate is recovered in a condensate collector.

The condensate recovered in the condensate collector comprises two phases: a water phase with a low concentration in n-butanol and an organic phase with a high concentration in n-butanol. The ratio between the 2 phases is strongly dependent on the temperature, the separation being more efficient at a lower temperature.
In one embodiment, the water phase in the condensate is distilled to recover the n-butanol of this low n-butanol concentrated phase.
The organic phase from the condensate may also be distilled to recover the n-butanol.
Alternatively, the n-butanol may be recovered from the condensate by other well-known means such as membrane separation technologies.
In one embodiment, the stripping gas which has been depleted in n-butanol is recycled to the fermentation broth.
Preferably, the recycling is continuous.
This recycling of n-butanol depleted stripping gas allows maintaining anaerobiosis and possibly also adjusting the redox potential.

In one embodiment, the method of the invention further comprises a step of feeding the fermentation broth with some culture medium during the fermentation, especially to compensate for the loss of fermentation broth volume.
This feeding may be continuous or sequential, since high glycerol amounts are not inhibitory. The feeding rate can be connected with the base consumption, which is a good indicator for 1,3-PDO production

In a further step of the method according to this invention, the 1,3-PDO is recovered from the fermentation broth.
In one embodiment, the step of recovering 1,3-PDO comprises a step of distillation of the fermentation broth.

Alternatively 1,3-PDO can be recovered after a filtration step to remove all microorganisms, and inorganic materials. A solvent extraction step can be designed to remove 1,3-PDO.

The invention will be further illustrated by the following figures and examples. However, these examples and figures should not be interpreted in any way as limiting the scope of the present invention.

### FIGURES

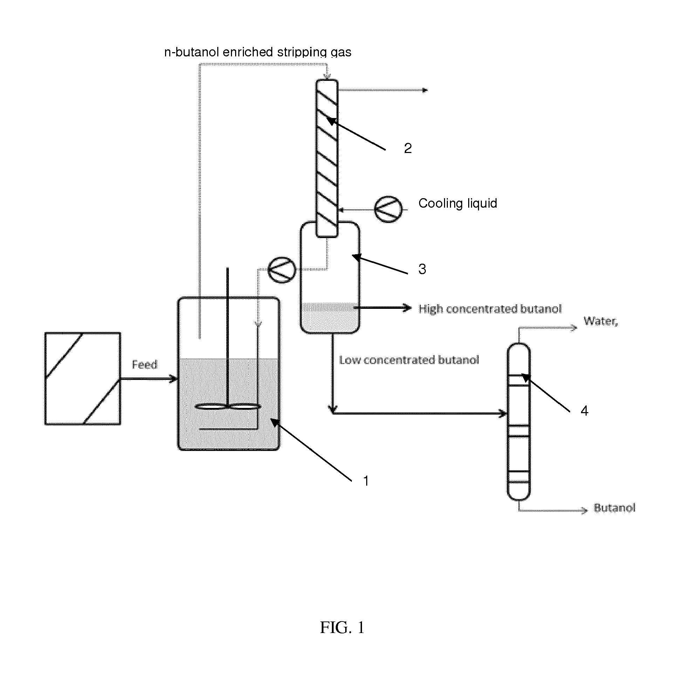

Figure 1 shows an apparatus for carrying out an embodiment of the invention.
This apparatus comprises a fermentor (1), a condenser (2), a condensate collector (3) and a distillation unit (4).
Figure 2 shows the fermentation of 1,3-PDO and n-butanol from pure glycerol with in situ gas stripping (0,8 L/ min. nitrogen, started at 25h).
Figure 3 shows the fermentation of 1,3-PDO and n-butanol from pure glycerol without gas stripping.
Figure 4 shows the fermentation of 1,3-PDO and n-butanol from a glycerol:glucose blend (1:1) with in situ gas stripping (0,8 L/ min. nitrogen, started at 25h).

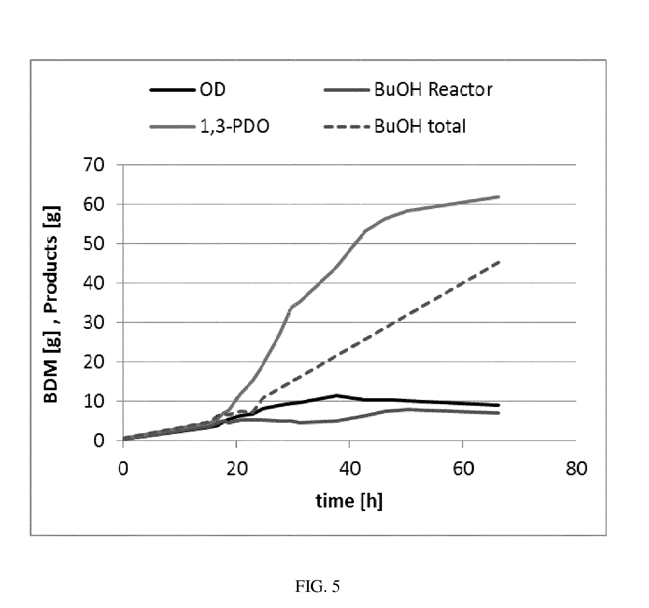

Figure 5 shows the fermentation of 1,3-PDO and n-butanol from pure glycerol with in situ gas stripping using the fermentation gases (10 L/min. nitrogen, started at 17h). Here the absolute amounts in [g] are given, resulting from 1,2 L fermentation broth.

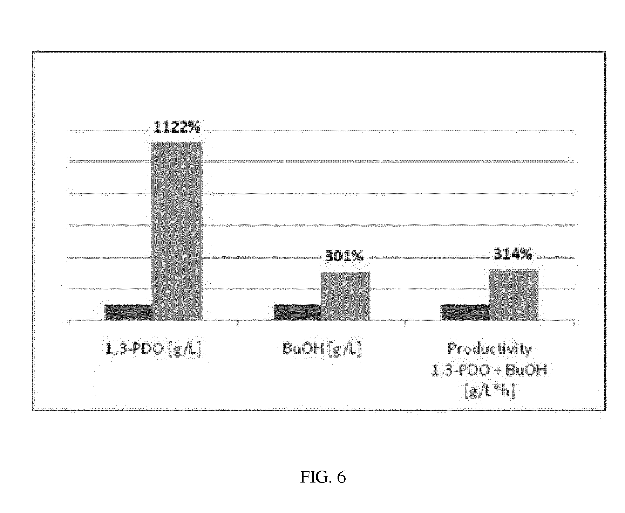

Figure 6 shows a comparison of products produced during simultaneous bioconversion of glycerol to butanol and 1,3-propanediol by *C. pasteurianum* by a conventional processes (on the left) or by the process of the invention (on the right).

### EXAMPLES

### Material and Methods:

### Medium composition

The medium composition is disclosed in the table below.

| **Glycerol as feedstock** | |
|---|---|
| Compound | g/L |
| Pure glycerol | 80 * |
| Yeast extract | 2 |
| KH₂PO₄ | 0.5 |
| K₂HPO₄ | 0.5 |
| (NH₄)2SO₄ | 5 |
| MgSO₄.7H₂O | 0.2 |
| CaCl₂.2H₂O | 0.02 |
| Cysteine | 0.5 |
| FeSO₄ | 0.015 |

| | |
|---|---|
| *start with 80 g/L. Further 200 g are fed in 3 equal portions, mixed with same amount of water. In example 2, glycerol is replaced with a mixture of glycerol/glucose with a ratio 1:1. | |

### Experimental procedure

The steps involved in the production of n-butanol are:
1) N2 was sparged at the beginning of fermentation to maintain anaerobic conditions.
2) Cultures were kept as spores or at -80°C cryocultures. Inocula were prepared by transferring to minimal medium and after scaling up in different reactors, the cells were inoculated into the fermentor (5-10% of the total liquid volume in fermentor).
3) Fermentations were run at 35°C, pH was kept constant at 6 by adding 2.5 M KOH. Within fermentation, butanol is separated from the broth by internal gas stripping. Own fermentation gases (and N2 at the beginning) were used as stripping gases. Gas stripping was started, when BuOH concentration reached ∼5 g/L.
4) The condensate (water +butanol + some ethanol) was further purified by a distillation process for further product improvement. The butanol / water mixture formed an azeotrope. The water phase contained roughly 70 g/L, and the organic phase up to 700 g/L of butanol, which facilitated the distillation process and should be considered.
5) The fermentation broth was distilled to get the accumulated 1,3-PDO.

### Reaction conditions

1) Reactor size: 2L, Working volume: 1.5L
2) Temperature: 35°C cultivation,
3) Reaction time: 66 h, incl. 50 h stripping
4) Gas flow for gas stripping ∼ 6.5 L / min per liter fermentation broth.

### Results:

### Example 1: Use of N2 for in situ gas stripping

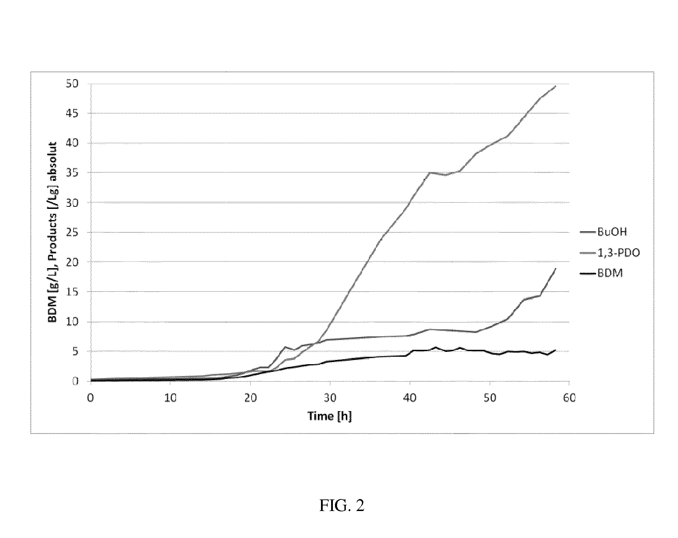

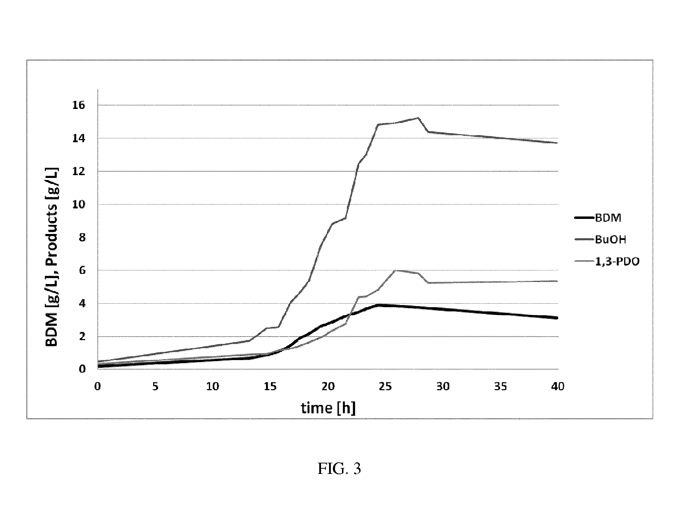

In a glycerol fermentation with *C. pasteurianum* DSM 525 and N₂ gas stripping, as high as 50 g 1,3-PDO/L and an accumulated concentration of 18 g/L butanol were reached in a single process (Fig.2). Such a high 1,3-PDO concentration can normally only be realized in fermentation processes producing only 1,3-PDO or a very small amount of butanol (<5 g/L). Without gas stripping, the C. *pasteurianum* strain produced less than 6 g/L 1,3-PDO and 14 g/L butanol under similar conditions (Fig.3).

The fermentation with gas stripping has also a much higher 1,3-PDO yield in this case, i.e. 0.28 g 1,3-PDO/g glycerol. In the comparative fermentation without gas stripping (Fig.3) the overall yield was 0.10 g 1,3-PDO / g glycerol only.

### Example 2: Production of 1,3-PDO and n-butanol from glycerol:glucose blend (1:1)

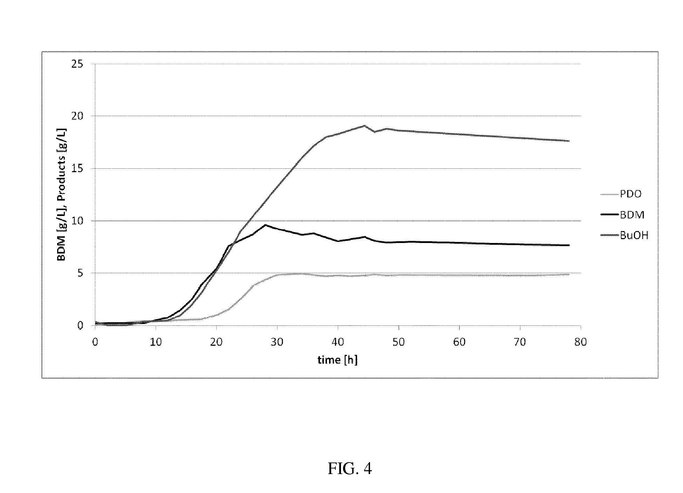

Glucose fermentation using natural microorganisms does not result in 1,3-PDO production. In a fermentation with *C. pasteurianum* grown on glycerol:glucose blend (1:1) with gas stripping, butanol (up to 18 g/L with a yield of 0.26 g butanol /g glycerol), and PDO (5 g/L with a yield of 0.10 g 1,3-PDO / g glycerol) (see Fig 4) could be obtained simultaneously.

### Example 3: Use of fermentation gases for in situ gas stripping

In this case, it was performed a glycerol fermentation with *C. pasteurianum* and internal gas stripping, using the fermentation gas (CO2, H2) itself as stripping gas. Here, as high as 54 g 1,3-PDO/L and an accumulated concentration of 39 g/L butanol has been reached. 7 g/L butanol remained in the fermentation broth, the remaining part was stripped out, giving a total amount of 46 g butanol produced in this 1.2 L batch (Fig.2). Compared to gas stripping with nitrogen, the overall yields could be further increased for butanol: to 0.19 g butanol/g glycerol. Compared to an experiment without gas stripping (Fig.3) (with an overall yield of both products of 0.38 g butanol+1,3-PDO /g glycerol) it was reached an overall yield of 0.45 g butanol+1,3-PDO /g glycerol in this example. This value could be further increased by the utilization of a combined substrate of glycerol and glucose.

### REFERENCES

Throughout this application, various references describe the state of the art to which this invention pertains. The disclosures of these references are hereby incorporated by reference into the present application.

Jensen TO, Kvist T, Mikkelsen MJ, Westermann P. Production of 1,3-PDO and butanol by a mutant strain of Clostridium pasteurianum with increased tolerance towards crude glycerol. AMB Express. 2012 Aug 17;2(1):44.
Jensen TO, Kvist T, Mikkelsen MJ, Christensen PV, Westermann P. Fermentation of crude glycerol from biodiesel production by Clostridium pasteurianum. J Ind Microbiol Biotechnol. 2012 May;39(5):709-17.
Biebl H. Fermentation of glycerol by Clostridium pasteurianum--batch and continuous culture studies.J Ind Microbiol Biotechnol. 2001 Jul;27(1):18-26. Xue C, Zhao J, Lu C, Yang ST, Bai F, Tang IC. High-titer n-butanol production by clostridium acetobutylicum JB200 in fed-batch fermentation with intermittent gas stripping. Biotechnol Bioeng. 2012 Nov;109(11):2746-56.

## Claims

1. A method for the anaerobic coproduction of 1,3-propanediol (1,3-PDO) and n-butanol comprising the successive steps of:
(a) culturing a *Clostridium* species strain in a fermentation broth comprising at least glycerol as a source of carbon, thereby producing n-butanol and 1,3-propanediol,
(b) recovering n-butanol and
(c) recovering 1,3-propanediol from the fermentation broth, **characterized in that**:
- a reducing agent other than H₂ is added to the fermentation broth and the fermentation broth is sparged with a gas selected from H₂, N₂, CO₂ and their mixtures, or
- the fermentation broth is sparged with a gas comprising CO₂, H₂ and N₂.

2. The method according to claim 1 **characterized in that** the *Clostridium* species is selected from the group consisting of *Clostridium pasteurianum, Clostridium* butyricum and *Clostridium diolis,* preferably *Clostridium pasteurianum.*

3. The method according to claim 1 or 2 **characterized in that** the reducing agent is cysteine.

4. The method according to any one claims 1 to 3 **characterized in that** the step of sparging the fermentation broth with gas comprises a step of gas stripping.

5. The method according to any one of claims 1 to 4 **characterized in that** the stripping gas used in the gas stripping step is the fermentation gas produced by culturing the *Clostridium* species.

6. The method according to any one of claims 1 to 5 **characterized in that** the step of removing n-butanol from the fermentation broth is started when the concentration of n-butanol in the fermentation broth reaches a value comprised between 4 and 6 g/L, preferably 5 g/L.

7. The method according to any one of claims 1 to 6 **characterized in that** the n-butanol is recovered by passing a flow of stripping gas through the fermentation broth so as to form a n-butanol enriched stripping gas.

8. The method according to claim 7 **characterized in that** the n-butanol is recovered from the enriched stripping gas by condensation in a condenser (2).

9. The method according to claim 8 **characterized in that** the condenser (2) is cooled from 0°C to room temperature, preferably from 0°C to 5°C, more preferably at 1°C by a cooling liquid.

10. The method according to any one of claims 1 to 9 **characterized in that** the step of recovering 1,3-PDO comprises a step of distillation of the fermentation broth.

11. The method according to any one of claims 1 to 10 **characterized in that** the pH of the fermentation broth is from 5 to 7, preferably from 5.5 to 6.5, more preferably 6.

12. The method according to any one of claims 1 to 11 **characterized in that** it comprises a step of feeding the fermentation broth with some culture medium during the fermentation.

13. The method according to any one of claims 1 to 12 **characterized in that** the fermentation broth is at a temperature from 34°C to 65°C, more preferably from 34°C to 37°C, most preferably at 35°C.
